(19) **Europäisches Patentamt / European Patent Office / Office européen des brevets**

(11) **EP 4 650 455 A1**

(12) # EUROPEAN PATENT APPLICATION
published in accordance with Art. 153(4) EPC

(43) Date of publication:
**19.11.2025 Bulletin 2025/47**

(21) Application number: **24741603.5**

(22) Date of filing: **12.01.2024**

(51) International Patent Classification (IPC):
*C12P 21/06* (2006.01)   *A23C 9/00* (2006.01)
*A23J 3/14* (2006.01)   *A23L 2/00* (2006.01)
*A23L 2/38* (2021.01)   *A23L 5/00* (2016.01)
*A23L 33/185* (2016.01)

(52) Cooperative Patent Classification (CPC):
**A23C 9/00; A23J 3/14; A23L 2/00; A23L 2/38;
A23L 5/00; A23L 33/185; C12P 21/06**

(86) International application number:
**PCT/JP2024/000708**

(87) International publication number:
**WO 2024/150831 (18.07.2024 Gazette 2024/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.01.2023 JP 2023003016**

(71) Applicant: **Amano Enzyme Inc.**
**Nagoya-shi**
**Aichi 460-8630 (JP)**

(72) Inventor: **SAKAI, Kiyota**
**Gifu 509-0109 (JP)**

(74) Representative: **Vossius & Partner**
**Patentanwälte Rechtsanwälte mbB**
**Siebertstraße 3**
**81675 München (DE)**

(54) **METHOD FOR PRODUCING PROCESSED OAT PROTEIN-CONTAINING LIQUID COMPOSITION**

(57)   The purpose of the present invention is to provide processing technology that improves the foamability of an oat protein-containing liquid composition. This method for producing a processed oat protein-containing liquid composition includes: an enzymatic treatment step for treating an oat protein-containing liquid composition with a protein deamidase at a prescribed pH, thereby obtaining a liquid composition that has been subjected to enzymatic treatment; and a pH lowering step for lowering the pH of the liquid composition that has been subjected to the enzymatic treatment, thereby obtaining a processed oat protein-containing liquid composition. This method makes it possible to improve the foamability of an oat protein-containing liquid composition.

EP 4 650 455 A1

**Description**

Technical Field

The present invention relates to processing technique for improving the foamability of an oat protein-containing liquid composition.

Background Art

Drinks rich in nutrients such as proteins have been widely familiar to people because they can easily take nutrients therefrom.

Against the background of a recent increase in the number of vegetarians, allergic problems, religious reasons, and the like, as an alternative to animal milk typified by cow's milk, drinks made from soybean, oat, almond, and the like rich in plant proteins have been widely spread.

On the other hand, when a milk protein raw material is replaced with a plant protein raw material, the milk protein raw material cannot be replaced as it is in many cases because the types of the proteins are different. For this reason, various techniques related to a processing treatment for modifying plant protein raw materials have been proposed.

For example, PTL 1 describes that the problem of high viscosity specific to an oat suspension is solved by treating an oat suspension with α-amylase and β-amylase, and proteins and β-glucans can be maintained.

PTL 2 describes that dispersion stability and solubility can be improved by treating a plant milk with a protein deamidase.

Citation List

Patent Literature

   PTL 1: US Patent No. 6,451,369
   PTL 2: WO 2022/071418 A

Summary of Invention

Technical Problem

Among plant protein materials, an oat is known as a foodstuff having high nutritional value. When oat milk is treated with a protein deamidase, a modification effect of improving foamability is obtained. However, in view of the recent spread of plant protein food or drink products, the foamability improving effect obtained merely by a treatment with a protein deamidase is not sufficient, and it is desired to further improve the modifying effect.

Thus, an object of the present invention is to provide a processing technique for improving the foamability of an oat protein-containing liquid composition.

Solution to Problem

As a result of intensive studies, the present inventor has unexpectedly found that by performing a treatment of an oat protein-containing liquid composition with a protein deamidase and then lowering the pH, the foamability of the resulting processed oat protein-containing liquid composition is improved as compared with the case where the treatment is not performed. The present invention has been accomplished by further studying based on this finding.

In summary, the present invention provides aspects of invention as itemized below.

Item 1. A method for producing a processed oat protein-containing liquid composition, the method including: an enzyme treatment step of treating an oat protein-containing liquid composition with a protein deamidase at a prescribed pH to obtain an enzyme-treated liquid composition; and a pH lowering step of lowering pH of the enzyme-treated liquid composition to obtain a processed oat protein-containing liquid composition.

Item 2. The method according to item 1, in which a value obtained by subtracting a pH of the processed oat protein-containing liquid composition at 25°C from the prescribed pH at 25°C is 0.2 to 4.

Item 3. The method according to item 1 or 2, in which the prescribed pH at 25°C is 5.0 to 9.0.

Item 4. The method according to any one of items 1 to 3, in which the processed oat protein-containing liquid composition has a pH of 4 to 7.5 at 25°C.

Item 5. The method according to any one of items 1 to 4, in which a content of an oat protein in the oat protein-containing liquid composition is 0.01 to 15 wt%.

Item 6. The method according to any one of items 1 to 5, in which the oat protein-containing liquid composition is oat milk.

Item 7. The method according to any one of items 1 to 6, in which the protein deamidase is protein glutaminase.

Item 8. The method according to any one of items 1 to 7, in which an amount of the protein deamidase used per 1 g of an oat protein contained in the oat protein-containing liquid composition is 0.05 U or more.

Item 9. An oat protein-containing food or drink product produced using a processed oat protein-containing liquid composition obtained by the method according to any one of items 1 to 8.

Item 10. The oat protein-containing food or drink product according to item 9, in which the processed oat protein-containing liquid composition is foamed. Advantageous Effects of Invention

According to the present invention, there is provided a processing technique for improving the foamability of an oat protein-containing liquid composition.

Description of Embodiments

1. Method for producing processed oat protein-containing liquid composition

The method for producing a processed oat protein-containing liquid composition of the present invention is characterized by including an enzyme treatment step of treating an oat protein-containing liquid composition with a protein deamidase at a prescribed pH to obtain an enzyme-treated liquid composition, and a pH lowering step of lowering the pH of the enzyme-treated liquid composition to obtain a processed oat protein-containing liquid composition. The processed oat protein-containing liquid composition obtained by the production method of the present invention has been subjected to processing to improve foamability as compared with the case where the pH lowering step is not performed. Furthermore, the foam stability of the oat protein-containing liquid composition is deteriorated through a treatment with a protein deamidase, and in a preferred embodiment of the processed oat protein-containing liquid composition obtained by the production method of the present invention, there is performed processing to improve foam stability as compared with the case where the pH lowering step is not performed. Hereinafter, the method for producing a processed oat protein-containing liquid composition of the present invention will be described in detail.

1-1. Enzyme treatment step

In the enzyme treatment step, an oat protein-containing liquid composition is treated with a protein deamidase at a prescribed pH to afford an enzyme-treated liquid composition. Specifically, an enzyme-treated liquid composition is obtained by preparing an oat protein-enzyme mixed liquid with the prescribed pH including an oat protein-containing liquid composition and a protein deamidase, and subjecting the mixture to a treatment to promote an enzymatic reaction.

1-1-1. Oat protein-containing liquid composition

The oat protein-containing liquid composition is not particularly limited as long as it is a liquid in which an oat protein is dissolved and/or dispersed in water. Examples of the oat protein-containing liquid composition include (i) liquids obtained by dispersing a dry powder (preferably oat flour) of a material containing an oat protein (preferably oat seeds, more preferably threshed oat) in water and, as necessary, removing an insoluble matter derived from a bran layer or the like of an oat seed by any means such as centrifugal filtration, filtration, a filter bag, or a sieve, (ii) liquids obtained by crushing and dispersing a material containing an oat protein (preferably oat seeds, more preferably threshed oat) in water and, as necessary, removing an insoluble matter derived from a bran layer or the like of an oat seed by any means such as centrifugal filtration, filtration, a filter bag, or a sieve, (iii) liquids in which the oat protein content is increased by, for example, removing a component other than the oat protein from a liquid of (i) or (ii) described above, and (iv) liquids obtained by dissolving and/or dispersing a dry powder prepared from any liquid among (i) to (iii) described above in water. In addition, the oat protein-containing liquid composition is preferably one prepared from the liquid of any one of (i) to (iv) described above by further treating the liquid with amylase. From the viewpoint of further enhancing the foamability of the processed oat protein-containing liquid composition or from the viewpoint of improving the foam stability in addition to that viewpoint, preferred examples of the oat protein-containing liquid composition include oat milk, which is an embodiment of an amylase treatment product of the liquid of the above (i), the liquid of the above (ii), or the above (iv) corresponding to either of those liquids.

The content of the oat protein in the oat protein-containing liquid composition to be used in the present invention is not particularly limited, and is, for example, 0.01 to 15 wt%, 0.1 to 7.5 wt%, or 0.25 to 5 wt%, and from the viewpoint of further enhancing the foamability of the processed oat protein-containing liquid composition or from the viewpoint of improving the foam stability in addition to that viewpoint, the content is preferably 0.5 to 3 wt%, or 0.75 to 2.5 wt%, and more preferably 1 to 2 wt%, 1.2 to 1.8 wt%, or 1.4 to 1.6 wt%.

When the oat protein-containing liquid composition to be used in the present invention is the liquid of the above (i), the liquid of the above (ii), or the liquid of the above (iv) corresponding to either of these liquids, the content of the oat protein material in the oat protein-containing liquid composition is not particularly limited, and is, for example, 0.1 to 50 wt%, 1 to 40 wt%, or 2 to 30 wt%, and from the viewpoint of further enhancing the foamability of the processed oat protein-containing liquid composition or from the viewpoint of improving the foam stability in addition to that viewpoint, the content is preferably 2.5 to 25 wt%, or 5 to 20 wt%, and more preferably 8 to 16 wt%, 9 to 14 wt%, or 10 to 12 wt%.

The oat protein-containing liquid composition may or may not contain any components as other components in addition to the oat protein and water. Examples of such other components include components derived from an oat seed, other food materials, and food additives. Examples of the food additive include a thickener, a binder, a seasoning, a pH adjuster, a buffering agent, a coloring agent, and a flavor.

1-1-2. Protein deamidase

The type, origin, etc. of the protein deamidase are not particularly limited as long as the enzyme exhibits an action of decomposing an amide group-containing side chain of a protein without cleaving a peptide linkage and without crosslinking the protein. Examples of the protein deamidase include protein deamidases derived from the genus Chryseobacterium, the genus Flavobacterium, the genus Empedobacter, the genus Sphingobacterium, the genus Aureobacterium, or the genus Myroides, the genus Luteimicrobium, the genus Agromyces, the genus Microbacterium, and the genus Leifsonia, which are disclosed in JP 2000-50887 A, JP 2001-218590 A, and WO 2006/075772 A. These protein deamidases may be used singly or two or more thereof may be used in combination.

Examples of the protein deamidase include protein glutaminase and protein asparaginase, and in a broad sense, protein arginine deaminase is also included. Among these protein deamidases, protein glutaminase is preferred from the viewpoint of further enhancing the foamability of the processed oat protein-containing liquid composition or from the viewpoint of improving the foam stability in addition to that viewpoint.

Among those protein deamidase enzymes, protein deamidases derived from the genus Chryseobacterium are preferable, protein glutaminases derived from the genus Chryseobacterium are more preferable, and protein glutaminases derived from Chryseobacterium proteolyticum species are still more preferable from the viewpoint of further enhancing the foamability of the processed oat protein-containing liquid composition or from the viewpoint of improving the foam stability in addition to that viewpoint.

The protein deamidase can be prepared from a culture solution of a microorganism as an origin of the above protein deamidase. A specific preparation method may be a method of collecting a protein deamidase from a culture solution or a bacterial cell of the above microorganism. For example, in the case of using a microorganism that secretes a protein deamidase, the enzyme can be separated and/or purified after bacterial cells are collected from the culture solution by filtration, centrifugation, or the like in advance, as necessary. In the case of using a microorganism that does not secrete a protein deamidase, after bacterial cells are collected from the culture solution in advance, as necessary, the bacterial cells are disrupted by a pressurization treatment, an ultrasonic treatment, or the like to expose the enzyme, and then the enzyme can be separated and/or purified. As an enzyme separation and/or purification method, a known protein separation and/or purification method can be used without particular limitation, and examples thereof include a centrifugal separation method, a UF concentration method, a salting-out method, and various chromatography methods using an ion exchange resin. The separated and/or purified enzyme can be powdered by a drying method such as freeze-drying or reduced-pressure drying, and can also be powdered using an appropriate excipient and/or drying aid in the drying method. The separated and/or purified enzyme can also be liquefied by adding an appropriate additive and subjecting it to filtration sterilization.

The amount of the protein deamidase to be used for the oat protein-containing liquid composition is not particularly limited, and the amount of the protein deamidase to be used per 1 g of the oat protein contained in the oat protein-containing liquid composition is, for example, 0.05 U or more, 0.1 U or more, or 0.5 U or more, and is preferably 1 U or more, 1.5 U or more, 2 U or more, or 2.5 U or more, more preferably 3 U or more, 3.5 U or more, or 4 U or more, and still more preferably 4.5 U or more, or 5 U or more from the viewpoint of further enhancing the foamability of the processed oat protein-containing liquid composition or from the viewpoint of improving the foam stability in addition to that viewpoint. The upper limit of the amount of the protein deamidase to be used per 1 g of the oat protein contained in the oat protein-containing liquid composition also is not particularly limited, and examples thereof include 500 U or less, 200 U or less, 100 U or less, 80 U or less, 60 U or less, 40 U or less, 30 U or less, 20 U or less, 12 U or less, 8 U or less, or 7 U or less.

For the activity of the protein deamidase, the amount of enzyme liberating 1 $\mu$mol of ammonia per minute using benzyloxycarbonyl-L-glutaminylglycine (Z-Gln-Gly) as a substrate is defined as 1 unit (1 U).

1-1-3. Reaction operation and treatment conditions

The method for preparing the oat protein-enzyme mixed liquid is not particularly limited. Examples thereof include a method of preparing an oat protein-enzyme mixed liquid by preparing an oat protein-containing liquid composition and then mixing the oat protein-containing liquid composition with a protein deamidase; and a method of preparing an oat protein-enzyme mixed liquid simultaneously with the preparation of an oat protein-containing liquid composition by mixing materials of the oat protein-containing liquid composition with a protein deamidase.

The specific range of the prescribed pH is not particularly limited as long as the effect of the present invention can be obtained, and can be appropriately set according to the optimum pH of the protein deamidase to be used. Specific examples of the prescribed pH (pH at 25°C) include 5.0 to 9.0 or 5.0 to 8.5, and from the viewpoint of further enhancing the foamability of the processed oat protein-containing liquid composition or improving the foam stability in addition to that viewpoint, the prescribed pH is preferably 5.5 to 8.5, more preferably 6 to 8.4, still more preferably 6.5 to 8.3, further preferably 7 to 8.2, and still further preferably 7.2 to 8.2, 7.4 to 8.2, 7.6 to 8.2, or 7.8 to 8.2.

Other treatment conditions (temperature, time, etc.) of the oat protein-enzyme mixed liquid are not particularly limited as long as the effect of the present invention can be obtained.

The treatment temperature can be appropriately set according to the optimum temperature of the protein deamidase to be used. Specific examples of the treatment temperature include 20 to 70°C, preferably 40 to 68°C, more preferably 50 to 66°C, still more preferably 55 to 64°C, and further preferably 58 to 62°C. The treatment time can be appropriately set according to the scale, the treatment temperature, etc. of the oat protein-enzyme mixed liquid. Specific examples of the treatment time include, for example, 0.5 to 4 hours, preferably 0.6 to 2 hours, and more preferably 0.7 to 1.5 hours, or 0.8 to 1.2 hours.

These treatment conditions may be determined through a preliminary experiment according to the required foamability of the processed oat protein-containing liquid composition or the degree of foamability and foam stability.

1-2. pH lowering step

In the pH lowering step, the pH of the enzyme-treated liquid composition obtained by the above-described enzyme treatment step is lowered to afford a processed oat protein-containing liquid composition.

Examples of the method for lowering the pH include a method of adding a pH adjuster, a method of mixing with an acidic food or drink product, and a method of adding an acidic food additive other than the pH adjuster. As a method for lowering the pH, any one of these methods may be used singly, or two or more methods may be used in combination. Examples of the pH adjuster include inorganic acids such as hydrochloric acid and sulfuric acid; organic acids such as lactic acid and citric acid; and salts of strong acids and weak bases. In addition, in order to finely adjust the pH, an inorganic base such as an alkali metal hydroxide (sodium hydroxide, potassium hydroxide, etc.); an organic base such as triethanolamine or triisopropanolamine may be used in combination. Among these pH adjusters, inorganic acids are preferred. Examples of the acidic food or drink product include oat yogurt, coffee, and fruit drink. Examples of these acidic food or drink products include unheated and uncooled food or drink products, or food or drink products cooled (for example, to a temperature higher than 5°C and lower than normal temperature, or 5°C or lower). Examples of the acidic food additive other than the pH adjuster include acidic amino acids and acidic vitamins. These pH adjusters, acidic food or drink products, and food additives may be used singly, or two or more of them may be used in combination.

The degree of lowering the pH may be appropriately set according to the degree of the foamability or the degree of the foamability and the foam stability required of the processed oat protein-containing liquid composition, and examples thereof include such a degree that the value obtained by subtracting the pH of the processed oat protein-containing liquid composition at 25°C from the prescribed pH at 25°C is, for example, 0.2 to 4, and from the viewpoint of further enhancing the foamability of the processed oat protein-containing liquid composition or from the viewpoint of improving the foam stability in addition to that viewpoint, preferably 1 to 4, more preferably 1.5 to 4, still more preferably 2 to 4, and further preferably 2.4 to 4, 2.6 to 3.5, or 2.8 to 3.2.

The pH at 25°C of the processed oat protein-containing liquid composition to be obtained in the pH lowering step is specifically, for example, 4 to 7.8, and from the viewpoint of further enhancing the foamability of the processed oat protein-containing liquid composition or from the viewpoint of improving the foam stability in addition to that viewpoint, preferably 4 to 7.5 or 4 to 7, more preferably 4 to 6.6, still more preferably 4 to 6.1, and further preferably 4 to 5.6. In general, the foamability and foam stability are remarkably deteriorated at low pH, but the processed oat protein-containing liquid composition obtained by the present invention is superior in foamability, or foamability and foam stability, and thus superior foamability or superior foamability and foam stability can be obtained even at low pH. From such a viewpoint, suitable examples of the pH at 25°C of the processed oat protein-containing liquid composition include 4 to 6.6, more preferably 4 to 6.1, still more preferably 4 to 5.6, and further preferably 4 to 5.4, 4.5 to 5.4, 4.5 to 5.2, 4.8 to 5.2, or 4.8 to 5.

1-3. Other steps

The method for producing the processed oat protein-containing liquid composition of the present invention may or may not include a step other than the above-described enzyme treatment step and the pH lowering step. Examples of the other step include a step of preparing an oat protein-containing liquid composition, a step of deactivating an enzyme, an enzyme treatment step with an enzyme other than a protein deamidase, a cooling step, a filtration step, and a drying step. These other steps may be performed singly or two or more steps may be performed in combination.

The step of preparing an oat protein-containing liquid composition can be performed by any method by which the oat protein-containing liquid composition can be prepared. Examples of such a method include (I) a method of preparing an oat protein-containing liquid composition by dispersing a dry powder (preferably oat flour) of a material containing an oat protein (preferably, oat seeds, more preferably threshed oat) in water and, as necessary, removing an insoluble matter derived from a bran layer or the like of an oat seed by any means such as centrifugal filtration, filtration, a filter bag, or a sieve, (II) a method of preparing an oat protein-containing liquid composition by crushing and dispersing a material containing an oat protein (preferably oat seeds, more preferably threshed oat) in water and, as necessary, removing an insoluble matter derived from a bran layer or the like of an oat seed by any means such as centrifugal filtration, filtration, a filter bag, or a sieve, (III) a method of preparing an oat protein-containing liquid composition from the liquid obtained by the method (I) or (II) by increasing the oat protein content by, for example, removing a component other than the oat protein, and (IV) a method of preparing an oat protein-containing liquid composition by drying a liquid obtained by any method among the above (I) to (III) and dissolving and/or dispersing the resulting dry powder in water.

When the oat protein-containing liquid composition is prepared as an amylase-treated liquid, the liquid obtained by any one of the above methods (I) to (IV) is treated with an amylase. Examples of the amylase include $\alpha$-amylase and $\beta$-amylase. These amylases may be used singly, or both of them may be used in combination. In the present invention, from the viewpoint of further enhancing the foamability of the processed oat protein-containing liquid composition or from the viewpoint of improving the foam stability in addition to that viewpoint, the oat protein-containing liquid composition is preferably one treated with $\alpha$-amylase and $\beta$-amylase.

The $\alpha$-amylase is not particularly limited, and examples thereof include $\alpha$-amylases derived from the genus Aspergillus (e.g., Aspergillus oryzae and Aspergillus niger); and the genus Bacillus (e.g., Bacillus amyloliquefaciens, Bacillus subtilis, and Bacillus licheniformis). An $\alpha$-amylase derived from the genus Bacillus is preferable, and an $\alpha$-amylase derived from Bacillus amyloliquefaciens species is more preferable.

The amount of $\alpha$-amylase to be used is, for example, 0.01 to 1000 U, preferably 0.1 to 100 U, more preferably 0.5 to 50 U, still more preferably 1 to 30 U, and further preferably 5 to 20 U, or 8 to 15 U per 1 g of the starch in the oat protein-containing liquid composition.

For the activity of $\alpha$-amylase, the amount of the enzyme that reduces coloring due to iodine by 10% per minute using potato starch as a substrate is defined as 1 unit (1 U).

The $\beta$-amylase is not particularly limited, and examples thereof include a $\beta$-amylase derived from a plant (wheat, soybean, etc.) and a $\beta$-amylase derived from the genus Bacillus. A $\beta$-amylase derived from the genus Bacillus is preferable, and a $\beta$-amylase derived from Bacillus flexus species is more preferable.

The amount of $\beta$-amylase to be used is, for example, 0.0001 to 9 U, preferably 0.001 to 0.9 U, more preferably 0.01 to 0.4 U, still more preferably 0.03 to 0.2 U, and further preferably 0.06 to 0.12 U per 1 g of the starch in the oat protein-containing liquid composition.

For the activity of $\beta$-amylase, the amount of the enzyme that leads to an increase in reducing power corresponding to 1 mg of glucose per minute using potato starch as a substrate is defined as 1 unit (1 U).

Conditions of the amylase treatment (treatment pH, treatment temperature, and treatment time) may be appropriately set according to the optimum pH and the optimum temperature of the enzyme used, the scale of the oat protein-containing liquid composition, the degree of required solubility, and the like.

For example, as to the treatment pH, the pH (at 25°C) of the oat protein-containing liquid composition before the amylase treatment is, for example, 5.5 to 7.5, and from the viewpoint of further enhancing the foamability, is preferably 6.2 to 7.2, more preferably 6.5 to 7.2, still more preferably 6.8 to 7.2, and particularly preferably 7.0, and from the viewpoint of further enhancing the foam stability, is preferably 6.2 to 7.2, more preferably 6.3 to 6.7, and particularly preferably 6.5.

The treatment temperature is, for example, 50 to 65°C, preferably 50 to 60°C, and more preferably 52 to 56°C. The treatment time is, for example, 0.5 to 2 hours.

In the enzyme deactivation step, the temperature condition and/or the pH condition for deactivating the enzyme used can be appropriately selected in consideration of the temperature characteristic and/or the pH characteristic of the processed oat protein-containing liquid composition, and preferably the temperature condition can be selected. Specific temperature conditions include, for example, 85 to 120°C, preferably 90 to 100°C, and more preferably 93 to 98°C, and the treatment time includes, for example, 1 to 30 minutes, or 1 to 10 minutes, and preferably 3 to 8 minutes.

2. Oat protein-containing food or drink product

The processed oat protein-containing liquid composition obtained by the production method described above can be used as a material for food or drink products (oat protein-containing food or drink products). Since the processed oat protein-containing liquid composition is superior in foamability, or foamability and foam stability, as a specific form of the oat protein-containing food or drink products, it can be used for food or drink products in a state in which the processed oat protein-containing liquid composition is foamed, or food or drink products which are to be provided for food or drink products by foaming the processed oat protein-containing liquid composition at the time of use.

Specific examples of the oat protein-containing food or drink product include oat milk, oat cream, oat yogurt, and food or drink products containing them. These food or drink products may be mixed with another drink or the like such as tea, coffee, or fruit drink, or may be mixed with the other drink at the time of use and provided for eating or drinking. In addition, since the processed oat protein-containing liquid composition exhibits superior foamability or superior foamability and foam stability even when the pH is relatively low, preferred forms of these food or drink products are those prepared as oat yogurt; those mixed with low-pH drinks such as coffee and fruit drinks; those to be mixed with the low-pH drinks at the time of use and provided for eating or drinking. The temperature of the food or drink product is not particularly limited, and may be any of a food or drink product heated (for example, higher than normal temperature and lower than 50°C, or 50°C or higher), an unheated and uncooled food or drink product, and a food or drink product cooled (for example, higher than 5°C and lower than normal temperature, or 5°C or lower).

Such a food or drink product can be produced by appropriately performing a seasoning step and/or a preparing step using the processed oat protein-containing liquid composition. The specific methods to be used in the seasoning step and the preparing step may be appropriately determined by those skilled in the art according to the food or drink product as the final form.

Examples

Hereinafter, the present invention will be specifically described with reference to Examples, but the present invention is not to be construed as being limited to the following Examples.

[A. Materials used and enzymes]

The following materials and enzymes were used.

[Table 1]

| Material or enzyme | Product name | Manufacturer |
|---|---|---|
| Protein deamidase | Protein glutaminase (PG) derived from Chryseobacterium proteolyticum | Amano Enzyme Inc. |
| α-amylase | α-amylase derived from Bacillus amyloliquefaciens | Amano Enzyme Inc. |
| β-amylase | β-amylase derived from Bacillus flexus | Amano Enzyme Inc. |
| Oat flour | Premium oat flour Protein content: 13.1 wt% Carbohydrate content: 67.7 wt% | Slow Food Kitchen |

[B. Method of measuring enzyme activity]

[B-1. Protein deamidase]

The protein deamidase activity value was measured by the following method.

To 1 mL of a 0.2 M phosphate buffer (pH 6.5) containing 30 mM Z-Gln-Gly, 0.1 mL of a sample solution containing protein deamidase was added, and the mixture was allowed to stand at 37°C for 10 minutes, and then 1 mL of a 0.4 M TCA solution was added to stop the reaction. As a blank, 1 mL of a 0.4 M TCA solution was added to 1 mL of a 0.2 M phosphate buffer (pH 6.5) containing 30 mM Z-Gln-Gly, and then 0.1 mL of a sample solution containing protein deamidase was further added, and the mixture was allowed to stand at 37°C for 10 minutes.

For the solution obtained as described above, the amount of ammonia generated in the reaction liquid was measured using Ammonia Test Wako (FUJIFILM Wako Pure Chemical Corporation). A calibration curve representing the relationship between the ammonia concentration and the absorbance (630 nm) was prepared using an ammonia standard solution (ammonium chloride), and from the calibration curve, the ammonia concentration in the reaction liquid was determined.

The amount of the enzyme that produces 1 μmol of ammonia per minute was defined as 1 unit (1 U), and the activity of the protein deamidase was calculated from the following formula. In the formula, the reaction liquid amount is 2.1, the enzyme solution amount is 0.1, and Df is a dilution rate of the enzyme solution. 17.03 is a molecular weight of ammonia.

Protein deamidase activity (U/mL) = ammonia concentration in reaction liquid (mg/L) × (1/17.03) × (reaction liquid amount/enzyme solution amount) × (1/10) × Df                           [Equation 1]

[B-2. α-amylase]

The α-amylase activity value was measured by the following method.

10 mL of the 1% potato starch substrate solution (0.1 mol/L acetic acid (pH 5.0)) was warmed at 37°C for 10 minutes, 1 mL of a sample solution containing α-amylase was then added, and the mixture was immediately shaken. This solution was left standing at 37°C for 10 minutes, then 1 mL of this solution was added to 10 mL of a 0.1 mol/L hydrochloric acid solution, and the mixture was immediately shaken. Next, 0.5 mL of this solution was weighed, and 10 mL of a 0.0002 mol/L iodine test solution (Japanese Pharmacopoeia) was added thereto. The mixture was shaken, and then absorbance (AT) at a wavelength of 660 nm was measured using water as a control. Separately, 1 mL of water was added instead of the sample solution, and the absorbance (AB) was measured. A 0.0002 mol/L iodine test solution (Japanese Pharmacopoeia) was prepared by adding 10 mL of water to 12.7 g of iodine and 25 g of potassium iodide, mixing the mixture well, then adding water to adjust the volume to 100 mL, and further diluting the mixture 2500 times with water. The amount of the enzyme at which the color development of potato starch by iodine was reduced by 10% per minute was defined as 1 unit (1 U).

[Equation 2]

$$\alpha\text{-amylase activity (U/g, U/mL)} = (AB - AT)/AB \times 1/W$$

AT: Absorbance of reaction solution
AB: Absorbance of blank liquid
W: Amount of sample in 1 mL of sample solution (g or mL)

[B-3. β-amylase]

The β-amylase activity value was measured by the following method.

A potato starch was used as a substrate, the potato starch was dried in advance at 105°C for 2 hours, 1.0 g of the dried product thereof was weighed, 20 mL of water was added, and 5 mL of a sodium hydroxide solution (2 mol/L) was gradually added with stirring to form a paste. Next, the paste was heated in a boiling water bath for 3 minutes with stirring, and then 25 mL of water was added. After cooling, the mixture was neutralized by adding a hydrochloric acid solution (2 mol/L) and a hydrochloric acid solution (0.1 mol/L), 10 mL of a 1 mol/L acetic acid-sodium acetate buffer solution (pH 5.0) was added, and water was further added to make 100 mL, thereby affording a substrate solution.

10 mL of the substrate solution was weighed and warmed at 37°C for 10 minutes, 1 mL of a sample solution containing β-amylase was added and immediately shaken, the mixture was warmed at the same temperature for 10 minutes, 4 mL of a Fehling's test solution was added and shaken lightly, the mixture was heated in a boiling water bath for 15 minutes, and then cooled to 25°C or lower, and 2 mL of a potassium iodide solution (a solution prepared by dissolving 30 g of potassium iodide in 70 mL of water) and 2 mL of sulfuric acid (1→6) were added, thereby affording a test solution. The Fehling's test solution was prepared at the time of use by mixing a copper solution prepared by weighing 34.66 g of fine crystals of copper(II) sulfate pentahydrate and adding water thereto to make 500 mL, and an alkaline tartrate solution prepared by weighing 173 g of (+)-potassium sodium tartrate tetrahydrate and 50 g of sodium hydroxide and adding water thereto to make 500 mL, in a ratio of 1 volume of the copper solution to 1 volume of the alkaline tartrate solution.

Separately, the same operation as in the preparation of the test solution was performed using 10 mL of water instead of the substrate solution to prepare a comparative solution. For the test solution and the comparative solution, liberated iodine was titrated with a 0.05 mol/L sodium thiosulfate solution. The end point was set to the time at which one or two drops of a soluble starch solution were added when the titration was close to the end point, and the resulting blue color disappeared. The amount of the enzyme that leads to an increase in reducing power corresponding to 1 mg of glucose per minute was defined as 1 unit (1 U), and the β-amylase activity was calculated from the following formula.

[Equation 3]

$$\beta\text{-amylase activity (U/g, U/mL)} = \text{amount of glucose (mg)} \times 1/10 \times 1/M$$

$$\text{Amount of glucose (mg)} = (b - a) \times 1.6 \times f$$

a: Titration value (mL) of enzyme reaction solution
b: Titration value (mL) of blank liquid
1.6: 1 mL of a 0.05 mol/L sodium thiosulfate solution corresponds to a glucose amount of 1.6 mg.
1/10: Unit conversion factor for reaction time (min)
M: Amount of sample in 1 mL of sample solution (g or mL)
f: Factor of 0.05 mol/L sodium thiosulfate solution (for quantitative determination)

[Oat protein-containing liquid composition]

An oat protein-containing liquid composition was prepared as follows.

18.1 g of oat flour and 150 g of water were mixed, and a suspension was prepared in a 200 mL Erlenmeyer flask. This suspension was adjusted such that the pH (at 25°C) was 6.5, 7.0, or 7.5, 10 U of α-amylase per 1 g of starch and 0.09 U of β-amylase per 1 g of starch were added, and the mixture was allowed to react at 54°C for 1 hour. Thus, an oat protein-containing liquid composition (oat milk) was obtained.

To the resulting oat protein-containing liquid composition was added a protein deamidase (PG) in an amount of 5 U per 1 g of protein, whereby the pH was adjusted to 8.0 (25°C), and then the mixture was allowed to react at 60°C for 1 hour. After the reaction, the enzymes were deactivated by a heat treatment at 95°C for 5 minutes. Thereafter, centrifugation was performed at 1,000 rpm for 1 minute, and a supernatant (enzyme-treated liquid composition) was collected.

The collected supernatant (enzyme-treated liquid composition) was adjusted such that the pH (at 25°C) was 5.0, 5.5, 6.0, 6.5, 7.0, 7.5, or 8.0, whereby a processed oat protein-containing liquid composition (processed oat milk) was prepared. As a pH adjuster, a high-concentration aqueous solution of NaOH or HCl was used, so that there was almost no increase in liquid amount due to pH adjustment.

Separately, the same procedure was carried out except that the enzyme treatment with the protein deamidase (PG) was not carried out, whereby a PG-untreated oat protein-containing liquid composition (PG-untreated oat milk) was prepared.

[C. Foamability and foam stability]

The processed oat milk and PG-untreated oat milk obtained were evaluated for foamability and foam stability under the following conditions.

[C-1. Foamability]

Each of 50 mL of the processed oat milk and the PG untreated oat milk was homogenized at 18,000 rpm for 30 minutes. Immediately thereafter, the homogenized liquid was transferred to a 100 mL graduated cylinder, the volume VF0 including foam was measured, and the foamability was calculated using the following calculation formula.

[Equation 4]

$$\text{Foamability (\%)} = 100 \times (\text{VF0} - 50)/(50)$$

Furthermore, where the foamability in the case where the pH of the processed oat milk and the PG-untreated oat milk was 8.0 (in the case without the pH lowering step) was defined to be 1, the relative value of the foamability at each pH was derived as "foamability improvement index 1". In addition, where the foamability of the PG-untreated oat milk at each pH was 1, the relative value of the foamability of the processed oat milk was derived as "foamability improvement index 2". The results are shown in Tables 2A and 2B, Tables 3A and 3B, and Tables 4A and 4B depending on the pH at the time of the amylase treatment.

[Table 2A]

| pH during amylase treatment: 6.5 | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|
| pH after adjustment | | pH 8.0 | pH 7.5 | pH 7.0 | pH 6.5 | pH 6.0 | pH 5.5 | pH 5.0 |
| PG-untreated oat milk | Foamability | 12 | 13 | 14 | 14 | 15 | 16 | 7.5 |
| | Foamability improvement index 1 | | 1.08 | 1.17 | 1.17 | 1.25 | 1.33 | 0.63 |

[Table 2B]

| pH during amylase treatment: 6.5 | | | | | | | | |
|---|---|---|---|---|---|---|---|---|
| | | Comparative Example 8 | Example 1 | Example 2 | Example 3 | Example 4 | Example 5 | Example 6 |
| pH after adjustment | | pH 8.0 | pH 7.5 | pH 7.0 | pH 6.5 | pH 6.0 | pH 5.5 | pH 5.0 |
| Processed oat milk | Foamability | 12 | 14 | 17 | 19 | 21 | 23 | 18.8 |
| | Foamability improvement index 1 | | 1.17 | 1.42 | 1.58 | 1.75 | 1.92 | 1.57 |
| | Foamability improvement index 2 | 1.00 | 1.08 | 1.21 | 1.36 | 1.40 | 1.44 | 2.51 |

As shown in the row "foamability improvement index 1" in Tables 2A and 2B, the processed oat milks (Examples 1 to 6) were improved in foamability by lowering the pH after the PG treatment. Furthermore, as shown in Table 2A, when the pH was lowered to 5.0, the foamability was remarkably deteriorated (Comparative Example 7); however, as shown in the row "Foamability improvement index 2" in Table 2B, the processed oat milk exhibited superior foamability even when the pH was lowered to 5.0 after the PG treatment (Example 6).

[Table 3A]

| pH during amylase treatment: 7.0 | | | | | | |
|---|---|---|---|---|---|---|
| | | Comparative Example 9 | Comparative Example 10 | Comparative Example 11 | Comparative Example 12 | Comparative Example 13 |
| pH after adjustment | | pH 8.0 | pH 7.5 | pH 7.0 | pH 6.0 | pH 5.5 |
| PG-untreated oat milk | Foamability | 12 | 12 | 14 | 15 | 15 |
| | Foamability improvement index 1 | | 1.00 | 1.17 | 1.25 | 1.25 |

[Table 3B]

| pH during amylase treatment: 7.0 | | | | | | |
|---|---|---|---|---|---|---|
| | | Comparative Example 14 | Example 7 | Example 8 | Example 9 | Example 10 |
| pH after adjustment | | pH 8.0 | pH 7.5 | pH 7.0 | pH 6.0 | pH 5.5 |
| Processed oat milk | Foamability | 12 | 15 | 18 | 22 | 23 |
| | Foamability improvement index 1 | | 1.25 | 1.50 | 1.83 | 1.92 |
| | Foamability improvement index 2 | 1.00 | 1.25 | 1.29 | 1.47 | 1.53 |

[Table 4A]

| pH during amylase treatment: 7.5 | | | | | | |
|---|---|---|---|---|---|---|
| | | Comparative Example 15 | Comparative Example 16 | Comparative Example 17 | Comparative Example 18 | Comparative Example 19 |
| pH after adjustment | | pH 8.0 | pH 7.5 | pH 7.0 | pH 6.0 | pH 5.5 |

(continued)

| pH during amylase treatment: 7.5 | | | | | | |
|---|---|---|---|---|---|---|
| | | Comparative Example 15 | Comparative Example 16 | Comparative Example 17 | Comparative Example 18 | Comparative Example 19 |
| PG-untreated oat milk | Foamability | 12 | 12 | 13 | 15 | 16 |
| | Foamability improvement index 1 | | 1.00 | 1.08 | 1.25 | 1.33 |

[Table 4B]

| pH during amylase treatment: 7.5 | | | | | | |
|---|---|---|---|---|---|---|
| | | Comparative Example 20 | Example 11 | Example 12 | Example 13 | Example 14 |
| pH after adjustment | | pH 8.0 | pH 7.5 | pH 7.0 | pH 6.0 | pH 5.5 |
| Processed oat milk | Foamability | 12 | 14 | 17 | 20 | 22 |
| | Foamability improvement index 1 | | 1.17 | 1.42 | 1.67 | 1.83 |
| | Foamability improvement index 2 | 1.00 | 1.17 | 1.31 | 1.33 | 1.38 |

Similarly, as shown in the row "foamability improvement index 1" in Tables 3A and 3B and Tables 4A and 4B, the processed oat milks (Examples 7 to 14) were improved in foamability by lowering the pH after the PG treatment.

[C-2. Foam stability]

Each of 50 mL of the processed oat milk and the PG untreated oat milk was homogenized at 18,000 rpm for 30 minutes. Immediately thereafter, the homogenized liquid was transferred to a 100 mL graduated cylinder, the volume VF0 including foam was measured. After 30 minutes, the volume VF30 including foam was measured in the same manner, and the foam stability was calculated using the following calculation formula.

[Equation 5]

$$\text{Foam stability } (\%) = 100 \times (\text{VF30/VF0})$$

Furthermore, where the foam stability in the case where the pH of the processed oat milk and the PG-untreated oat milk was 8.0 was defined to be 1, the relative value of the foam stability at each pH was derived as "foam stability improvement index 1". In addition, where the foam stability of the PG-untreated oat milk at each pH was defined to be 1, the relative value of the foam stability of the processed oat milk was derived as "foam stability improvement index 2". The results are shown in Tables 5A and 5B and Tables 6A and 6B depending on the pH during the amylase treatment.

[Table 5A]

| pH during amylase treatment: 6.5 | | | | | | | | |
|---|---|---|---|---|---|---|---|---|
| | | Comparative Example 1 | Comparative Example 2 | Comparative Example 3 | Comparative Example 4 | Comparative Example 5 | Comparative Example 6 | Comparative Example 7 |
| pH after adjustment | | pH 8.0 | pH 7.5 | pH 7.0 | pH 6.5 | pH 6.0 | pH 5.5 | pH 5.0 |
| PG-untreated oat milk | Foam stability | 98 | 99 | 99 | 99 | 99 | 99 | 94.7 |
| | Foam stability improvement index 1 | | 1.01 | 1.01 | 1.01 | 1.01 | 1.01 | 0.97 |

EP 4 650 455 A1

[Table 5B]

| pH during amylase treatment: 6.5 | | | | | | | | |
|---|---|---|---|---|---|---|---|---|
| | | Comparative Example 8 | Example 1 | Example 2 | Example 3 | Example 4 | Example 5 | Example 6 |
| pH after adjustment | | pH 8.0 | pH 7.5 | pH 7.0 | pH 6.5 | pH 6.0 | pH 5.5 | pH 5.0 |
| Processed oat milk | Foam stability | 90 | 94 | 96 | 99 | 99 | 99 | 96.8 |
| | Foam stability improvement index 1 | | 1.04 | 1.07 | 1.10 | 1.10 | 1.10 | 1.08 |
| | Foam stability improvement index 2 | 0.92 | 0.95 | 0.97 | 1.00 | 1.00 | 1.00 | 1.02 |

As shown in Tables 5A and 5B, the processed oat milk was deteriorated in foam stability by the PG treatment (Comparative Example 8), but the foam stability was improved by lowering the pH after the PG treatment (Examples 1 to 6). The degree of the improvement in foam stability was equal to or greater than that in the case of no PG treatment when the pH was reduced to 6.5 or less (Examples 3 to 6). Furthermore, as shown in Table 5A, when the pH was lowered to 5.0, the foam stability was deteriorated (Comparative Example 7), but as shown in the row "foam stability improvement index 2" in Table 5B, the processed oat milk exhibited superior foam stability even when the pH was lowered to 5.0 after the PG treatment, and the degree thereof was greater than that in the case of no PG treatment (Example 6).

[Table 6A]

| pH during amylase treatment: 7.0 | | | | | | |
|---|---|---|---|---|---|---|
| | | Comparative Example 9 | Comparative Example 11 | Comparative Example 21 | Comparative Example 12 | Comparative Example 13 |
| pH after adjustment | | pH 8.0 | pH 7.0 | pH 6.5 | pH 6.0 | pH 5.5 |
| PG-untreated oat milk | Foam stability | 98 | 99 | 99 | 99 | 99 |
| | Foam stability improvement index 1 | | 1.01 | 1.01 | 1.01 | 1.01 |

[Table 6B]

| pH during amylase treatment: 7.0 | | | | | | |
|---|---|---|---|---|---|---|
| Example | | Comparative Example 14 | Example 8 | Example 15 | Example 9 | Example 10 |
| pH after adjustment | | pH 8.0 | pH 7.0 | pH 6.5 | pH 6.0 | pH 5.5 |
| Processed oat milk | Foam stability | 92 | 96 | 100 | 100 | 99 |
| | Foam stability improvement index 1 | | 1.04 | 1.09 | 1.09 | 1.08 |
| | Foam stability improvement index 2 | 0.94 | 0.97 | 1.01 | 1.01 | 1.00 |

Similarly, as shown in Tables 6A and 6B, the processed oat milk was deteriorated in foam stability by the PG treatment (Comparative Example 14), but the foam stability was improved by lowering the pH after the PG treatment (Examples 8 to 10 and 15), and the degree of the improvement of the foam stability was equal to or greater than that of the case of no PG treatment when the pH was lowered to 6.5 or less (Examples 9, 10, and 15).

**Claims**

1. A method for producing a processed oat protein-containing liquid composition, the method comprising:

   an enzyme treatment step of treating an oat protein-containing liquid composition with a protein deamidase at a prescribed pH to obtain an enzyme-treated liquid composition; and
   a pH lowering step of lowering pH of the enzyme-treated liquid composition to obtain a processed oat protein-containing liquid composition.

2. The method according to claim 1, wherein a value obtained by subtracting a pH of the processed oat protein-containing liquid composition at 25°C from the prescribed pH at 25°C is 0.2 to 4.

3. The method according to claim 1, wherein the prescribed pH at 25°C is 5.0 to 9.0.

4. The method according to claim 1, wherein the processed oat protein-containing liquid composition has a pH of 4 to 7.5 at 25°C.

5. The method according to claim 1, wherein a content of an oat protein in the oat protein-containing liquid composition is 0.01 to 15 wt%.

6. The method according to claim 1, wherein the oat protein-containing liquid composition is oat milk.

7. The method according to claim 1, wherein the protein deamidase is protein glutaminase.

8. The method according to claim 1, wherein an amount of the protein deamidase used per 1 g of an oat protein contained in the oat protein-containing liquid composition is 0.05 U or more.

9. An oat protein-containing food or drink product produced using a processed oat protein-containing liquid composition obtained by the method according to claim 1.

10. The oat protein-containing food or drink product according to claim 9, wherein the processed oat protein-containing liquid composition is foamed.

## INTERNATIONAL SEARCH REPORT

| International application No. |
| --- |
| **PCT/JP2024/000708** |

**A. CLASSIFICATION OF SUBJECT MATTER**

*C12P 21/06*(2006.01)i; *A23C 9/00*(2006.01)i; *A23J 3/14*(2006.01)i; *A23L 2/00*(2006.01)i; *A23L 2/38*(2021.01)i; *A23L 5/00*(2016.01)i; *A23L 33/185*(2016.01)i
FI:  C12P21/06; A23L33/185; A23L2/38 J; A23L2/00 S; A23C9/00; A23J3/14; A23L5/00 E; A23L5/00 J

According to International Patent Classification (IPC) or to both national classification and IPC

**B. FIELDS SEARCHED**

Minimum documentation searched (classification system followed by classification symbols)

C12P21/06; A23C9/00; A23J3/14; A23L2/00; A23L2/38; A23L5/00; A23L33/185

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2020/171106 A1 (AMANO ENZYME INC.) 27 August 2020 (2020-08-27) claims, examples | 1-10 |
| X | WO 2022/045152 A1 (AMANO ENZYME INC.) 03 March 2022 (2022-03-03) claims, examples | 1-10 |
| A | JP 2022-521426 A (AMANO ENZYME INC.) 07 April 2022 (2022-04-07) claims, examples | 1-10 |

☐ Further documents are listed in the continuation of Box C.    ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **02 April 2024** | **09 April 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/000708**

| Patent document cited in search report | | | Publication date (day/month/year) | Patent family member(s) | | | Publication date (day/month/year) |
|---|---|---|---|---|---|---|---|
| WO | 2020/171106 | A1 | 27 August 2020 | EP | 3928628 | A1 | |
| | | | | claims, examples | | | |
| WO | 2022/045152 | A1 | 03 March 2022 | EP | 4201216 | A1 | |
| | | | | claims, examples | | | |
| JP | 2022-521426 | A | 07 April 2022 | EP | 3930478 | A1 | |
| | | | | claims, examples | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6451369 B
- WO 2022071418 A
- JP 2000050887 A
- JP 2001218590 A
- WO 2006075772 A